# EUROPEAN PATENT APPLICATION

(11) **EP 1 445 000 A1**
(43) Date of publication of application: **11.08.2004**
(21) Application number: 02780066.3
(22) Date of filing: 11.11.2002
(51) Int. Cl.: A61M 5/32

(54) **INJECTION NEEDLE RETRACTABLE INJECTOR**

(30) Priority: 12.11.2001 JP 2001346025
(71) Applicant: JMS Co., Ltd., Hiroshima-shi, Hiroshima 730-8652 (JP)
(72) Inventor: KUNISHIGE, Takahiko, Hiroshima-shi, Hiroshima 731-0137 (JP)
(74) Representative: Schwarzensteiner, Marie-Luise, Dr.
(86) International application number: PCT/JP2002/011714
(87) International publication number: WO 2003/041771

(57) **Abstract**

An injection needle retractable injector, comprising: a barrel having a plunger inserted into a bore formed therein; a needle base attached to the tip part of the barrel; a hub attached to a bore of the needle base so as to be capable of being separated only toward the rear end; and an injection needle with a rear end part fixed to the tip part of the hub, the plunger being capable of being moved to the tip part of the barrel to engage the rear end part of the hub with the tip part of the plunger, so that the injection needle can be pulled into the barrel through the plunger; wherein the hub is held in the needle base by a fitting structure formed between the outer peripheral surface of the tip part of the hub and the inner surface of the tip part of the needle base, and in the fitted state in which the hub is positioned at the closest to the tip side of the needle base, the tip of the hub is positioned near or forward of the tip of the needle base in the axial direction.

## Description

### Technical Field

The present invention relates to a safe injection needle of a retractable injector type having a function for preventing accidental needlesticks. More specifically, it relates to an improvement of a fitting structure between a hub to which an injection needle is fixed and a needle base for attaching an injection needle to an injector tip part.

### Background Art

Examples of an injector having a function for preventing accidental needlesticks include an injector having a configuration in which an injection needle is pulled into a barrel (outer cylinder) after use. This type of injector is disclosed in, for example, JP 7(1995)-51372A or JP 11(1999)-342200A. The injectors described in these documents have a mechanism for engaging a hub to which an injection needle is fixed with a plunger (inner cylinder) and allows the hub to engage the plunger after use, thereby pulling the injection needle into the barrel via the plunger.

In the injector disclosed in JP11(1999)-342200A, in addition to an appropriate fitting strength between a hub with an injection needle and a needle base, a liquid sealing property (liquid shield performance) by the contact between the outer surface of the hub and the inner surface of the needle base should be maintained. In order to keep this liquid sealing property well, it is desirable that a contacting portion between the outer surface of the hub and the inner surface of the needle base is made not to be a surface having a broad area that is long in the axial direction but a surface having a thin circular area in the axial direction that is approximately linear. However, it is found that such a configuration is a negative factor from the viewpoint that the fitting strength increases as the contacting area is larger.

Furthermore, when the hub and the needle base are fitted to each other on the linear contacting part as in the above-mentioned configuration, although a predetermined fitting strength is kept at the time of manufacturing, the fitting strength is likely to be reduced according to the state of transportation or storage, and the risk of the hub being separated from the needle base is increased. Therefore, in the structure of the above-mentioned injection needle retractable injector, it is extremely difficult to provide and maintain both the liquid sealing property and the fitting strength.

Furthermore, when a conventional injection needle retractable injector such an injector as disclosed in JP11-342200A is manufactured, a problem that is difficult to solve is caused as follows: Figure 6 shows one example of a cross-sectional shape of an injection needle attaching structure 21 constituting an injection needle retractable injector of a conventional example.

The injection needle attaching structure 21 includes a hub 22, a needle base 23 and an injection needle 24. A tip part of the hub 22 is fitted to the rear part of a bore of the needle base 23; and a rear end part of the injection needle 24 is fixed to a tip part bore 22a of the hub 22. The bore of the needle base 23 includes a tip part bore 23a, a central part of a bore 23b and a rear part of a bore 23c. To the central part bore 23b, the tip part of the hub 22 is fitted and held. With the step formed at the boundary between the tip part bore 23a and the central part of a bore 23b, the hub 22 is restrained from moving toward the tip side and thus positioning is carried out The hub 22 has a pair of engaging legs 25 in the rear part, and each engaging leg 25 has a pulling protrusion 25a protruding outward is formed at the tip thereof.

The injection needle attaching structure 21 is attached to the tip of a barrel (not shown) so as to construct an injector. In this state, the engaging legs 25 are located in the barrel. A plunger attached slidably in the barrel has an engaging portion capable of being engaged with the pulling protrusion 25a. Via the engagement, by the operation of extracting the plunger toward the rear side of the barrel, the hub 22 is pulled into the barrel.

In the above-mentioned configuration, since the hub 22 has to be attached to the needle base 23 from the rear end, a method of attaching the injection needle 24 and the hub 22 to the needle base 23 is limited. In general, firstly, it is thought that the injection needle 24 is fixed to the hub 22, then the hub 22 with the injection needle 24 is inserted into the needle base 23 from the rear end, and the hub 22 is fitted to the needle base 23.

However, in this method, the tip of the injection needle 24 has to pass through the tip part bore 23a, and at this time, a needle edge may be damaged. Furthermore, the inner surface of the needle base 23 is scraped by the needle edge of the injection needle 23, and a burr, etc. (deep scraping or scraped residues) may be generated. Furthermore, since in general, injectors are manufactured by automatic assembly process, in order to insert the needle tip of the injection needle 24 into the inner surface of the needle base 23 without bringing it into contact with the inner surface, an automatic assembly device is required to have a considerable accuracy. For the above-mentioned reason, it is difficult to employ the above-mentioned method.

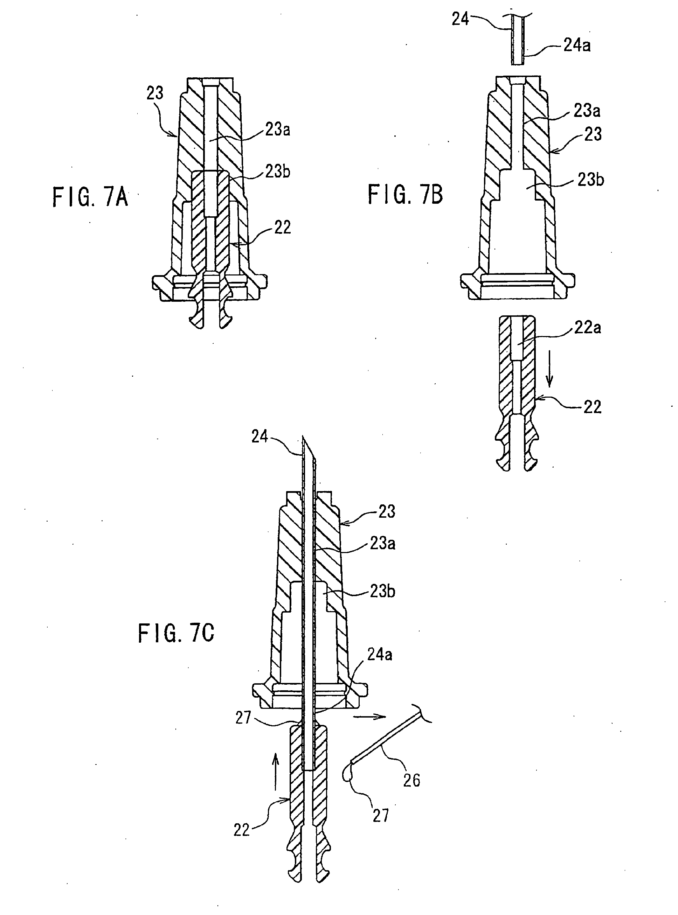

Therefore, in order to manufacture this injector, the steps shown in Figures 7A to 7C were required.

Firstly, as shown in Figure 7A, a hub 22 is fitted into a needle base 23 and the fitted structure is supplied to the following step. Then, as shown in Figure 7B, before an injection needle 24 is attached to the hub 22, the hub 22 is separated from the needle base 23 and moved in the direction of the rear side so as to form a state in which the tip of the hub 22 is exposed from the rear end of the needle base 23. In this state, the rear end part 24a of the injection needle 24 is inserted into the needle base 23 from the tip, and then, as shown in Figure 7C, the injection needle 24 is inserted into the tip part bore 22a of the hub 22 which is exposed from the rear end of the needle base 23. Then, an adhesive 27 is applied to the tip part of the exposed hub 22 by using an adhesive applicator 26 positioned at the side part of the hub 22. Then, the hub 22 to which the injection needle 24 is fixed is moved toward the tip side of the needle base 23 and fitted to the central part bore 23b of the needle base 23. According to this method, since the injection needle 24 is inserted into the needle base 23 from the rear end part 24a, the damage of the needle edge of the injection needle 24 is avoided.

However, as is apparent from Figures 7A to 7C, the above-mentioned method has a complicated step and takes much time and labor. Furthermore, in order to prevent separation of the injection needle 24 from the hub 22, or contrarily, to prevent the generation of failure in fixing between the needle base 23 and the hub 22 due to excess adhesive agent, the accuracy in applying adhesives is required so as to apply only to a predetermined region of the hub 22 without excess and deficiency. Thus, this method also was difficult to apply to an automatic assembly apparatus.

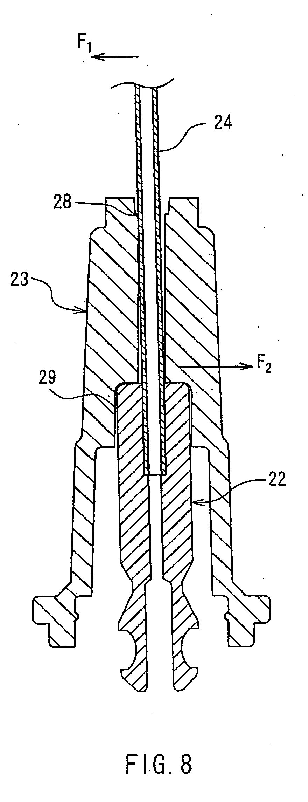

Furthermore, in the above-mentioned retractable injector, in order to increase the insertion allowance (compliance) of the injection needle 24 with respect to the tip part bore 23a of the needle base 23, the diameter of the tip part bore 23a is set to be larger than the outer diameter of the injection needle 24. Therefore, as shown in Figure 8, when a lateral force F1 is applied to the injection needle 24, the injection needle 24 swings in the lateral direction and is brought into contact with the tip part of the needle base 23. This contacting point works as a fulcrum 28, and thereby a lateral force F2 is applied from the rear end part 24a of the injection needle 24 to a fitting site 29 between the needle base 23 and the hub 22 in the central part bore 23b.

Even if the displacement of the injection needle 24 by the force F1 is small, since the fitting site 29 between the hub 22 and the needle base 23 is distant from the fulcrum 28, the displacement of the hub 22 due to force F2 in the fitting site 29 is increased. Therefore, rattling of the injection needle 24 at the tip part of the needle base 23 becomes an important factor for weakening the fitting between the hub 22 and the needle base 23. Thus, in the conventional retractable injectors, due to the structure in which the fitting site 29 between the hub 22 and the needle base 23 resides in the central part bore 23b that is in a deep place of the needle base 23, and is distant from the tip of the needle base 23 that works as a fulcrum, the fitting state between the hub 22 and the needle base 23 is difficult to be maintained.

### SUMMARY OF THE INVENTION

Therefore, it is an object of the present invention to provide an injection needle retractable injector that can be manufactured easily. It is also an object of the present invention to suppress the effect of an external force applied to the fitting between a hub and a needle base so as to maintain the fitting strength.

The injection needle of the present invention includes: a barrel having a plunger inserted into a bore formed therein; a needle base attached to the tip part of the barrel; a hub attached to a bore of the needle base so as to be capable of being separated only toward the rear end side; and an injection needle with a rear end part fixed to the tip part of the hub. The plunger is capable of being moved to the tip part of the barrel to engage the rear end part of the hub with the tip part of the plunger, so that the injection needle can be pulled into the barrel through the plunger In order to solve the above-mentioned problems, the hub is held in the needle base by a fitting structure formed between the outer peripheral surface of the tip part of the hub and the inner surface of the tip part of the needle base, and in the fitted state in which the hub is positioned at the closest to the tip side of the needle base, the tip of the hub is positioned near or forward of the tip of the needle base in the axial direction.

### BRIEF DESCRIPTION OF THE DRAWINGS

Figure 1A is a partial cross-sectional view showing an injection needle retractable injector according to one embodiment of the present invention.
Figure 1B is a cross-sectional view showing an operation of the same injection needle retractable injector.
Figure 1C is a cross-sectional view showing an operation of the same injection needle retractable injector.
Figure 2 is a cross-sectional view showing an injection needle attachment structure constituting the injector shown in Figure 1.
Figure 3A is a cross-sectional view showing a hub constituting the injection needle attachment structure shown in Figure 2.
Figure 3B is a cross-sectional view showing a needle base constituting the injection needle attachment structure shown in Figure 2.
Figure 4A is a cross-sectional view showing an enlarged section taken on line A-A of Figure 2.
Figure 4B is a cross-sectional view showing an enlarged section taken on line B-B of Figure 2.
Figure 5 is a cross-sectional view showing a manufacturing process of the injector shown in Figure 1.

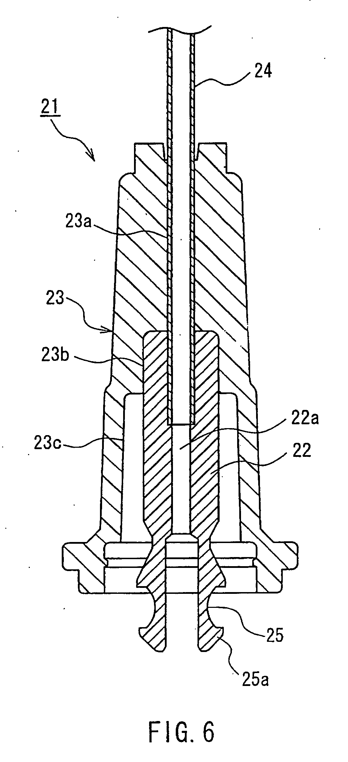

Figure 6 is a cross-sectional view showing an injection needle attachment structure constituting a conventional injection needle retractable injector.
Figures 7A to 7C are schematic views showing the procedures of manufacturing the injection needle attachment structure shown in Figure 6.
Figure 8 is a schematic view for explaining a problem of the injection needle attachment structure shown in Figure 6.

### BEST MODE FOR CARRYING OUT THE INVENTION

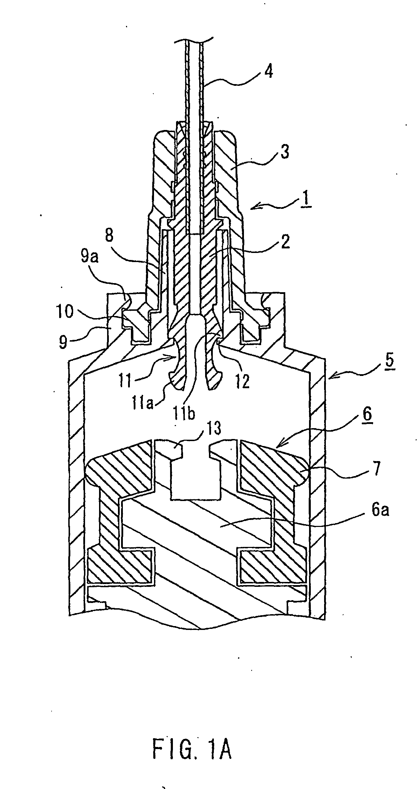

Hereinafter, the embodiment of the present invention will be described with reference to drawings. Figure 1A is a cross-sectional view, partially abbreviated, showing an injection needle retractable injector according to one embodiment.

Reference numeral 1 denotes an injection needle attachment structure and includes a hub 2 having a substantially cylindrical shape and being fitted to a bore of a substantially cylindrical shaped needle base 3; and an injection needle 4 fixed to the tip part of the hub 2. The hub 2 can be separated from the needle base 3 only toward the rear end side. This injection needle attachment structure 1 is attached to a barrel 5 so as to construct an injector by coupling the rear end part of the needle base 3 to the tip part of the substantially cylindrical-shaped barrel 5. Reference numeral 6 denotes a plunger inserted into the bore of the barrel 5. The plunger 6 includes a plunger main body 6a and a gasket 7.

At the tip part of the barrel 5, an inner wall 8 and an outer wall 9 are formed. Between the inner wall 8 and the outer wall 9, the rear end part of the needle base 3 is inserted. A latch part 9a formed on the inner side of the tip of the outer wall 9 is engaged with a coupling part 10 formed on the rear end of the needle base 3. Thereby, the attachment of the injection needle attachment structure 1 with respect to the barrel 5 is held.

The hub 2 has engaging legs 11 at the rear end part. Each of the engaging legs 11 has a pulling protrusion 11a formed at the tip part and a holding protrusion 11b formed at the side part. When the holding protrusion 11b is engaged with the hub holding part 12 provided on the tip part of the barrel 5, the hub 2 is restrained from moving toward the rear end side with respect to the needle base 3. The hub holding part 12 may be provided in the rear end part of the needle base 3. At the tip part of the plunger main body 6a, an engagement part 13 capable of engaging with the pulling protrusion 11a is provided.

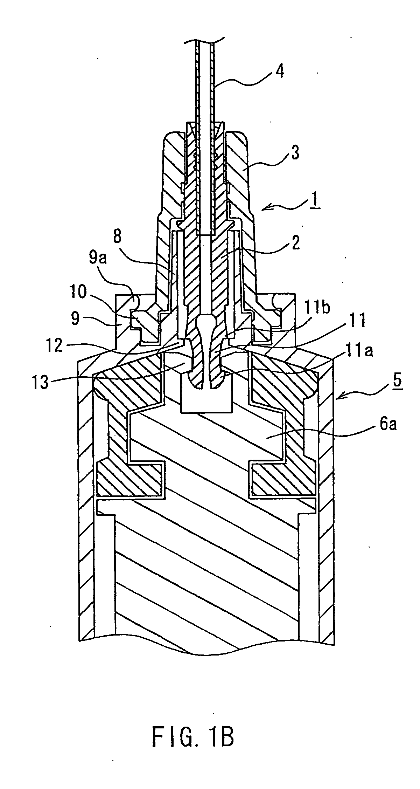

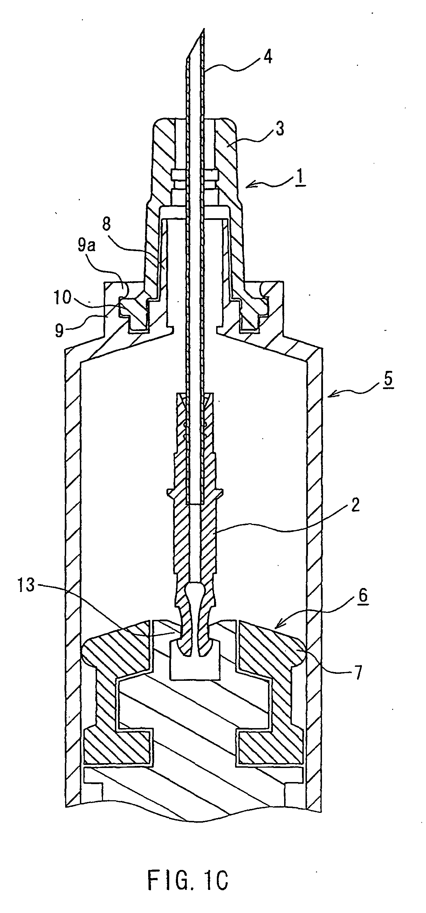

Figures 1B and 1C show an operation of the injection needle retractable injector, respectively. As shown in Figure 1B, when the plunger 6 is moved to the front end of the barrel 5, the pulling protrusions 11a of the engaging legs 11 enter the engagement part 13 of the plunger main body 6a. Thus, the engaging legs 11 are engaged with the plunger main body 6a while the engaging legs 11 are deformed so as to release the engagement of the holding protrusion 11b and the hub holding portion 12. Therefore, as shown in Figure 1C, when the plunger 6 is moved toward the rear end side of the barrel 5, the hub 2 is released form the engagement with the needle base 3, and thereby the injection needle 4 can be pulled into the barrel 5. By completely pulling the tip of the injection needle 4 into the barrel 5, a state of preventing accidental needlesticks can be obtained.

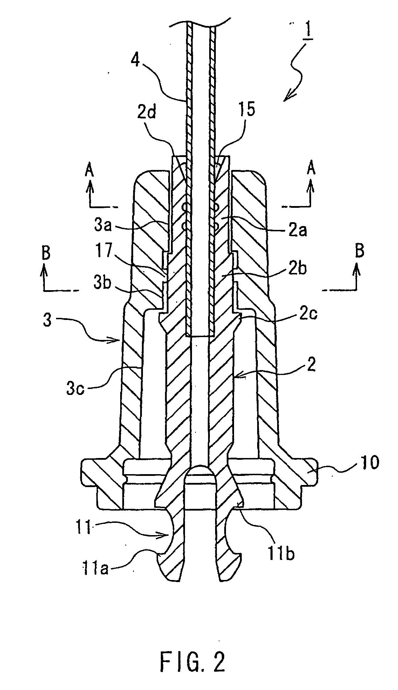

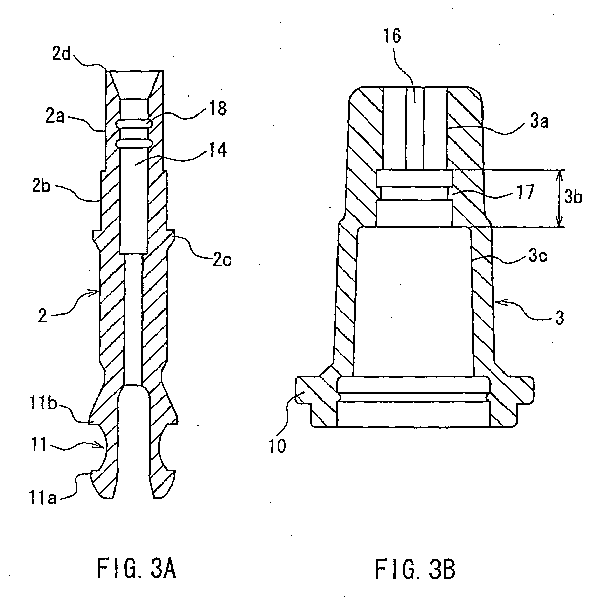

Then, the injection needle attachment structure 1 that is a characterizing portion of the present invention will be described in detail. Figure 2 is a cross-sectional view in the axial direction showing the injection needle attachment structure 1. Figure 3A is a cross-sectional view showing a hub 2, and Figure 3B is a cross-sectional view showing a needle base 3.

The injection needle attachment structure 1 has a fitting structure formed on the outer surface of the tip part of the hub 2 and on the inner surface of the tip part of the needle base 3. Thus, the hub 2 is held by the needle base 3. The fitting structure includes a fitting maintaining region provided in the front part in the axial direction and a liquid sealing securing region provided in the rear part. The fitting maintaining region is formed by the outer surface of the tip part 2a of the hub and a tip part bore 3a of the needle base 3. The liquid sealing securing region is formed by the outer surface of the hub central part 2b and the central part of the bore 3b of the needle base 3. A base end of the injection needle 4 is inserted into a needle fixing bore 14 formed on the tip side of the hub 2 and fixed thereto with an adhesive 15.

On the rear end part of the hub 2 located in the rear part bore 3c of the needle base 3, a position limiting protrusion 2c is provided. When the position limiting protrusion 2c is brought into contact with a step part formed on the boundary of the central part bore 3b and the rear part bore 3c of the needle base 3, the hub 2 is restrained from moving toward the tip side and thus the position is limited. Therefore, the hub 2 can be separated only toward the rear end side of the needle base 3. As shown in Figure 2, in a fitted state in which the hub 2 is located nearest to the tip side, the tip 2d of the hub 2 is exposed from the tip of the needle base 3.

The tip 2d of the hub may be recessed slightly from the tip end of the needle base 3. In a word, the hub tip 2d may be located in the vicinity of the tip of the needle base 3. The vicinity of the tip of the needle base 3 means the location approximating to the tip of the needle base 3 to such an extent that an adhesive applicator can be inserted easily. If in a condition of fitting the hub 2 and the needle base 3, the recessed amount of the tip 2d from the tip of the needle base 3 is within about 3 mm, it may be allowable practically. When the tip 2d is protruded from the tip of the needle base 3, the allowable range of the above-mentioned meaning is not limited. However, the protruding amount is set appropriately in the range in which general use is not hindered.

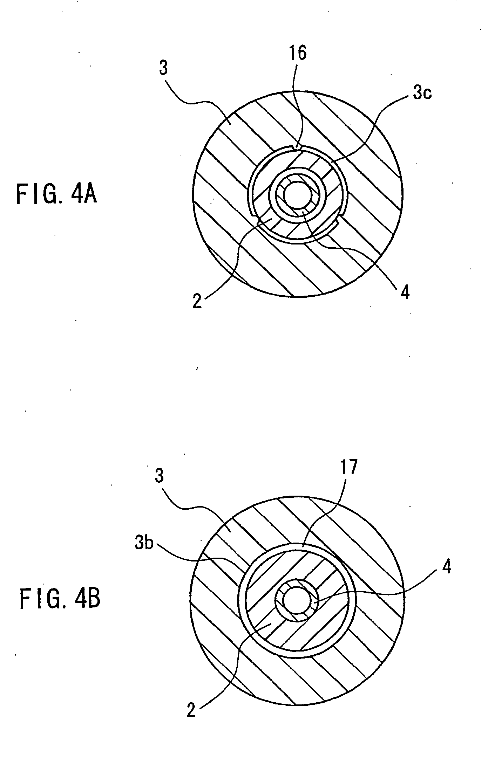

As shown in Figure 3B, on the inner surface of the tip part bore 3a of the needle base 3 forming a fitting maintaining region, longitudinal ribs 16 are formed. In the inner surface of the central part bore 3b of the needle base 3 forming a liquid sealing securing region, an annular protrusion 17 is formed. Figure 2 shows a state in which the annular protrusion 17 is brought into contact with the outer surface of the hub 2. Furthermore, Figure 4A is a cross-sectional view showing an enlarged section taken on line A-A of Figure 2. Figure 4B is a cross-sectional view showing an enlarged section taken on line B-B of Figure 2. As shown in Figure 4A, three ribs 16 are provided in the peripheral direction and each of the ribs 16 is disposed at an equal interval.

In the configuration mentioned above, by pressing force between the three ribs 16 and the outer surface of the hub tip part 2a, fitting of the hub 2 and the needle base 3 is maintained substantially. Furthermore, due to a dose attachment between the annular protrusion 17 and the outer surface of the hub central part 2b, the liquid sealing between the needle base 3 and the hub 2 can be secured.

The outer surface of the hub central portion 2b forms a gentle taper in which the diameter is increased toward the rear side. Therefore, the contact between the needle base 3 and the hub 2 in the liquid sealing securing region is achieved between the annular protrusion 17 formed on the inner surface of the needle base 3 and the outer surface of the hub 2 on which taper is formed. Thus, it is possible to fit the needle base 3 with the hub 2 smoothly. That is to say, since the annular protrusion 17 of the needle base 3 can slide smoothly on the tapered outer surface of the hub 2, the liquid sealing property can be secured easily and reliably. This taper is formed only on the outer surface of the hub central part 2b but is not formed on the outer surface of the hub tip part 2a. Furthermore, if necessary a taper may be formed on the inner surface of the needle base 3 in the axial direction and the annular protrusion may be provided on the outer surface of the hub 2.

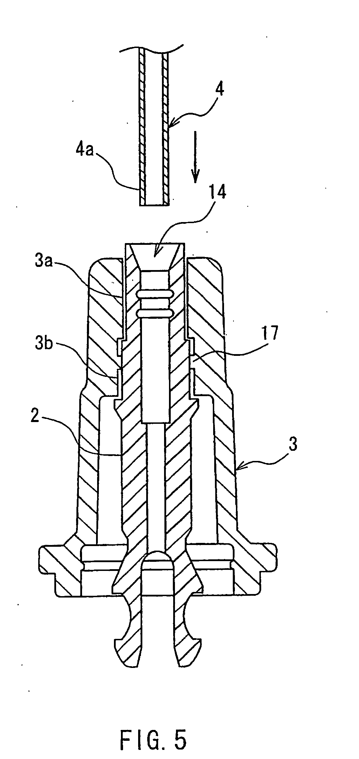

An example of a method for manufacturing the injection needle attachment structure having the above-mentioned configuration will be described with reference to Figure 5. Firstly, the hub 2 is inserted from the rear side of the needle base 3, so that the hub tip part 2a and the hub central part 2b are fitted to the tip part bore 3a and the central part bore 3b of the needle base 3. Thus the state shown in Figure 5 is achieved. Then, the injection needle base end 4a is inserted into a needle fixing hole 14 of the hub tip 2d exposed from the tip of the needle base 3 and fixed by using an adhesive agent 15 as shown in Figure 2. Alternatively, without using the adhesive agent 15, fixing may be completed by only inserting due to the fitting between the needle fixing hole 14 and the injection needle proximal end 4a.

By using the above-mentioned configuration and manufacturing method, the manufacturing process of the injection needle attachment structure 1 can be simplified. The reason follows. Firstly, in the fitting structure between the hub 2 and the needle base 3 of the present invention, with respect to the tip part bore 3a and the central part of the bore 3b of the needle base 3, the allowance for insertion of the injection needle 4 is not required to be considered. This is because when the hub 2 and the needle base 3 are fitted to each other, the hub tip 2d is located in the vicinity of the tip of the needle base 3, therefore when the injection needle 4 is inserted into the hub 2, it is not brought into contact with the inner surface of the needle base 3. When the injection needle 4 is embedded in the hub 2, as shown in Figure 5, the base end 4a of the injection needle can be inserted directly into the needle fixing hole 14 seen from the hub tip 2d that is exposed from the tip of the needle base 3 or recessed slightly. Thus, the steps can be carried out easily. Furthermore, because the injection needle 4 may not be inserted into the needle base 3, the accuracy required for automatically assembling apparatus can be alleviated and manufacturing process can be simplified.

In the case of the injection needle attachment structure 1 having the above-mentioned configuration, the following other manufacturing method can be used Firstly, the injection needle base end 4a is inserted and fixed in the needle fixing hole 14 at the hub tip 2d Next, the hub 2 with the injection needle is inserted into the needle base 3 from the rear side. That is to say the tip of the injection needle 4 is inserted from the rear end bore 3c, allowed to pass through the tip part bore 3a and to be exposed from the needle base 3. Furthermore, the hub 2 is pushed therein until the tip part 2a of the hub and the tip part bore 3a of the needle base 3 are fitted to each other. The hub 2 with the injection needle may be manufactured by a so-called insert molding, that is, by inserting the hub 2 at the time of molding. By attaching the hub 2 with injection needle produced by the inset molding to the needle base 3, complicated steps shown in Figures 7A to 7C can be more simplified.

Such a simple method can be carried out assuming the structure in which the hub tip part 2a is fitted to the tip part bore 3a of the needle base 3. The reason follows. In order to obtain such a fitting structure, it is necessary that the tip part bore 3a of the needle base 3 is substantially the same as the outer diameter of the hub tip part 2a. As a result, the diameter of the tip part bore 3a is larger than the outer diameter of the injection needle 4 by the dimension corresponding to the wall thickness of the hub 2 and sufficiently larger than the outer diameter of the injection needle 4. Therefore, when the injection needle 4 is inserted into the bore of the needle base 3, the possibility of the inner surface of the needle base 3 being damaged by the tip of the injection needle 4 is small. Thus, the manufacturing method as mentioned above can be used easily.

For example, the maximum diameter of the injection needle 4 used for the retractable injector is 1.25 mm or less. The inner diameter of the inner wall 8 of the tip of the barrel is preferably in the range from 2.7 mm to 3.0 mm in order to enable the attachment to a general purpose injection needle. The preferable range of the outer diameter of the hub tip part 2a assumed from the maximum diameter of the injection needle 4 and the inner diameter of the inner wall 8 of the barrel tip is in the range from 2.2 mm to 2.9 mm. The preferable range of the outer diameter of the tip part bore 3a of the needle base 3, which is calculated from the range of the outer diameter of the hub tip part 2a is in the range from 2.25 mm to 2.95 mm. Therefore, the diameter of the tip part bore 3a is sufficiently larger than the maximum diameter of the injection needle 4.

Herein, the diameter of the tip part bore 3a represents a diameter of the part in which the rib 16 is not formed. The diameter including the top part of the rib 16 is desired to be substantially the same or less of the outer diameter of the hub tip part 2a. Therefore, in the case where the rib 16 is not formed, the diameter of the tip part bore 3a is made to be substantially the same or less of the outer diameter of the hub tip part 2a.

According to the above-mentioned configuration of the injection needle attachment structure 1, the fitting force between the needle base 3 and the hub 2 can be maintained effectively. Various factors are relevant to maintaining the fitting force. Among them, it is important to prevent rattling of the injection needle 4 due to the lateral swinging as described in the conventional example. The simplest method for solving the problem is to locate the fitting site of the hub 2 and the needle base 3 at the tip side of the needle base 3. When considering that the tip part of the needle base 3 works as a fulcrum, when the injection needle 4 swings, it is found that if the fitting site is made dose to the tip of the needle base 3, the pressure to the fitting site caused by the lateral swing of the injection needle 4 can be decreased. In the injection needle attachment structure 1, since the fitting maintaining region is formed by the outer surface of the hub tip part 2a and the tip part bore 3a of the needle base 3, the reduction in the fitting strength between the needle base 3 and the hub 2 can be suppressed effectively

Furthermore, the above-mentioned configuration is effective in the improvement of the fitting force. The closeness at the fitting site between the needle base and the hub contributes much to improvement of the fitting force. Unlike the liquid sealing, the closeness at the fitting site herein denotes a state in which the needle base and the hub are brought into contact with each other securely so that the fitting site therebetween does not rattle. As mentioned above, in the fitting structure between the hub 2 and the needle base 3 of this embodiment, with respect to the tip part bore 3a of the needle base 3 and the central part bore 3b of the needle base 3, the allowance for insertion of the injection needle 4 is not required to be considered. Consequently, the diameter of the tip part bore 3a of the needle base 3 and the central part bore 3b are determined by considering only the fitting between the hub 2 and the outer surface. Therefore, rattling in fitting practically can be avoided by selecting, a dimension so that the hub 2 is brought into dose contact with the tip end bore 3a and the central part bore 3b.

The contact between the needle base and the hub is not limited to the entire contact but may be a partial contact as long as secure contact state in each contacting point is obtained. Therefore, as shown in Figure 4A, a plurality of longitudinal linear ribs 16 may be formed on the inner surface of the needle base 3 so as to employ fitting obtained by the contact between these ribs 16 and the outer surface of the hub tip part 2a. This fitting does not provide a liquid sealing property but is efficient in obtaining the secure fitting force. As shown in Figure 4A, although an area of one of the ribs 16 that is brought into contact with the outer surface of the hub 2 is small, the outer surface of the hub 2 is uniformly held by three ribs 16 from the periphery, and thus sufficient fitting strength can be obtained. Furthermore, the fitting by the contact between the plurality of ribs formed in the axial direction and the outer surface of the hub can be operated more easily as compared with the fitting by the contact on the entire periphery.

The number of ribs to be formed is preferably 2 to 4 when considering the cost, labor and effect into account. Note here that in the inner surface of the tip part bore 3a forming the fitting maintaining region, instead of longitudinal line ribs 16, a protrusion or protruding portion having the other shapes may be formed. Any protrusion or protruding portion having the other shape can exert the same effect as that of the ribs 16 as long as they cause appropriate pressure contact force between them and the outer surface of the hub tip part 2a. Furthermore, such protrusions or protruding parts formed on the outer surface of the hub tip part 2a can provide the same function.

Furthermore, the configuration of this embodiment is effective in coupling with both the fitting strength between the hub and the needle base and the liquid sealing property. In other words, since the fitting structure between the hub 2 and the needle base 3 is configured so as to be separated into the fitting maintaining region and the liquid sealing securing region, each region can be constructed in an optimum structure. In the above-mentioned configuration, vertical linear ribs 16 are used for the fitting maintaining region and annular protrusion 17 is used for the liquid sealing securing region, thereby achieving both functions. Hereinafter, this will be described specifically.

In order to obtain a sufficient fitting strength, it is necessary to increase the contact area in the entire fitting site. However, only the increase in the contacting area of the fitting portion may hinder the liquid sealing property between the hub and the needle base. Therefore, it is important to increase the contact area of the fitting portion while the liquid sealing property is secured. Therefore, sep aration of the fitting site is efficient. That it to say, by providing the fitting maintaining region and the liquid sealing securing region separately, the fitting maintaining region is configured so that a predetermined fitting strength is secured without considering the liquid sealing property and the liquid sealing securing region can be secured in a narrow annular region without considering the fitting strength.

In the above-mentioned configuration, it is preferable that the fitting maintaining region is formed closer to the tip side of the needle base as compared with the liquid sealing securing region. Thus, as mentioned above, the fitting site can be disposed in the needle base tip. side and the effect for maintaining the fitting force can be obtained efficiently. Furthermore, since the liquid sealing property is maintained at rear end side of the needle base, dead volume of drug solution in the bore of the needle base in the injector can be reduced. It is preferable that the fitting maintaining region is located at 0 mm to 5 mm rear part from the tip end of the needle base in the axial direction of the injection needle.

It is possible to prevent the decrease of the fitting strength due to the force applied via the injection needle mentioned above by providing a plurality of fitting maintaining regions in the axial direction. At this time, providing too many fitting sites, which are fitting maintaining region, is not significant, and occasionally, the liquid sealing property may be damaged. The number of the fitting sites is up to 2-3.

Furthermore, in order to increase the closeness of the fitting site without lowering the liquid sealing property in the liquid securing region, the fitting between the hub and the needle base preferably is obtained by the contact between the protruding shape formed on the inner surface of the needle base or the opposing surface. For example as shown in Figures 2, 3 and 4B, the fitting by the contact between the rib 16 formed on the inner surface of the needle base 3 and the annular protrusion 17 with respect to the outer surface of the hub 2 is efficient. With such a configuration, as mentioned above, although a contacting area of one of the ribs is small, since a plurality of contacting sites are dispersed, total fitness can be obtained sufficiently. Furthermore, the fitting site in the fitting maintaining region is located separately from the liquid sealing securing region and further a contacting area per protruding shape is small. Therefore, the fitting property in the fitting maintaining region has a small effect on the fitting state in the liquid sealing securing region with a low possibility of hindering the liquid sealing in the liquid sealing region.

Then, referring to Figure 3, the shape and dimension of the hub 2 will be described briefly. The tip side inner surface of the needle fixing hole 14 formed on the hub 2 is formed in a taper shape in which the inner diameter is increased toward the tip side so that the injection needle 4 can be inserted easily. Furthermore, near the tip of the needle fixing hole 14, two annular grooves 18 are engraved in order to hold the injection needle 4 more firmly. Furthermore, as mentioned above, on the outer surface of the hub central part 2b, a tapered portion for contacting annular protrusion is formed. The taper is formed in the range from 0 to 4.0 mm from the tip of the hub central part 2b. As mentioned above, the outer surface of the hub tip part 2a is a straight wall (not tapered). Furthermore, at the boundary between the outer surface of the hub tip part 2a and the outer surface of the hub central part 2b, a step is formed. The step together with the position limiting protrusion 2c prevents the hub 2 from moving to the tip side.

Next, shape and dimension of the needle base 3 will be described briefly. The tip part bore 3a and central part bore 3b of the needle base 3 is regulated by the outer diameter of the hub 2 basically. However, since the outer surface of central part 2b of the hub is provides with a taper, the diameter is changed slightly along the axial direction. The rib 16 is formed in the location 1 to 5.0 mm distant from the tip of the needle base 3 in a longitudinal linear shape. The annular protrusion 17 is formed at the location 2 mm to 7 mm distant from the tip of the needle base 3 in the width of 0.3 mm to 3.0 mm. The rear part bore 3c of the needle base 3 is formed with a sufficiently large diameter, so that the hub 2 easily is inserted fiom the rear side.

It is preferable that the fitting strength between the hub 2 and the needle base 3 is designed so that the force applied to the contacting part is in the range from 5 N/cm² to 15 N/cm². In this range, the hub 2 and the needle base 3 are not likely to be accidentally separated from each other before use and after use, a user can release the fitting easily.

### Industrial applicability

According to the injection needle retractable injector of the present invention, since the tip of the hub is located in the vicinity of the needle base, the manufacturing process can be simplified and the cost can be reduced. Even in an automatic assembling apparatus, since the accuracy that was necessary to conventional methods is not required, a general-purposed apparatus can be used. Furthermore, a manufacturing method capable of protecting an edge of an injection needle can be applied.

Furthermore, since the fitting site between the hub and the needle base is located at the tip side of the needle base, it is possible to decrease the pressing force applied to the fitting site due to the lateral swinging of the injection needle. Thus, the reduction in the fitting strength between the needle base and the hub can be suppressed effectively, so that the fitting force can be maintained for a long time.

## Claims

1. An injection needle retractable injector, comprising:
a barrel having a plunger inserted into a bore formed therein;
a needle base attached to the tip part of the barrel;
a hub attached to a bore of the needle base so as to be capable of being separated only toward the rear end; and
an injection needle with a rear end part fixed to the tip part of the hub,
the plunger being capable of being moved to the tip part of the barrel to engage the rear end part of the hub with the tip part of the plunger, so that the injection needle can be pulled into the barrel through the plunger;
wherein the hub is held in the needle base by a fitting structure formed between the outer peripheral surface of the tip part of the hub and the inner surface of the tip part of the needle base, and in the fitted state in which the hub is positioned at the closest to the tip side of the needle base, the tip of the hub is positioned near or forward of the tip of the needle base in the axial direction.

2. An injection needle retractable injector, comprising:
a barrel having a plunger inserted into a bore formed therein;
a needle base attached to the tip part of the barrel;
a hub attached to a bore of the needle base so as to be capable of being separated only toward the rear end; and
an injection needle with a rear end part fixed to the tip part of the hub,
the plunger being capable of being moved to the tip part of the barrel to engage the rear end part of the hub with the tip part of the plunger, so that the injection needle can be pulled into the barrel through the plunger;
wherein the diameter of a tip part bore of the needle base is substantially the same as or larger than the outer diameter of the tip part of the hub, and smaller than the inner diameter of the tip part of the barrel, and in the fitted state in which the hub is positioned at the closest to the tip side of the needle base, the tip of the hub is positioned near or forward of the tip of the needle base in the axial direction.

3. The injection needle retractable injector according to claim 1 or 2, wherein in the fitted state in which the hub is positioned at the closest to the tip side of the needle base, the tip of the hub is in substantially the same position as the tip of the needle base in the axial direction.

4. The injection needle retractable injector according to claim 1 or 2, wherein in the fitted state in which the hub is positioned at the closest to the tip side of the needle base, the tip of the hub is exposed from the tip of the needle base or retracted from the tip of the needle base.

5. The injection needle retractable injector according to claim 1 or 2, wherein the fitting structure is formed by the contact of the protruding shape formed on one of the inner surface of the needle base and the outer surface of the hub with the other opposing surface.

6. The injection needle retractable injector according to claim 5, wherein the fitting structure comprises a fitting maintaining region and a liquid sealing securing region, which are provided separately, the fitting maintaining region has a function of maintaining the fitting between the needle base and the hub, and the liquid sealing securing region has a function for securing the liquid sealing property between the needle base and the hub.

7. The injection needle retractable injector according to claim 6, wherein the fitting maintaining region is formed at a position closer to the tip side of the needle base than the liquid sealing securing region.

8. The injection needle retractable injector according to claim 6 or 7, wherein the fitting structure in the fitting maintaining region is formed by the contact between a plurality of ribs formed in the longitudinal direction of the inner surface of the needle base and the outer surface of the hub.

9. The injection needle retractable injector according to any one of claims 6 to 8, wherein the fitting structure in the liquid sealing securing region is formed by an annular protruding part formed on the inner surface of the needle base and a taper of the outer surface of the hub whose diameter becomes larger from the tip end to the rear end side in the axial direction; and the liquid sealing property is secured by the contact between the annular protruding portion and the outer surface of the hub.

10. The injection needle retractable injector according to any one of claims 1 to 9, wherein the hub with an injection needle that was insert-molded is attached to the needle base.

11. The injection needle retractable injector according to claim 1 or 2, wherein the hub has engaging legs at the rear end part, each engaging leg has a pulling protrusion formed at the tip part and holding protrusion formed at the side part, and the pulling protrusion can be engaged with the tip part of the plunger; when the holding protrusion is engaged with the tip part of the barrel or a hub holding part provided on the rear end part of the needle base, the hub is restrained form moving toward the rear end side of the needle base; and when the pulling protrusion is engaged with the tip part of the plunger, the engagement between the holding protrusion and the hub holding portion is released.

12. The injection needle retractable injector according to claim 1, wherein force applied to a part where the hub and the needle base are brought into contact with each other is in the range from 5 N/cm² to 15 N/cm².

13. An injection needle attachment structure for constructing an injection needle retractable injector comprising a hub attached to a bore of a needle base so as to be separated only toward the rear side, and an injection needle with a rear end part fixed to the tip part of the hub, wherein
the needle base can be attached to the tip part of a barrel having a plunger inserted into a bore formed therein;
the hub is provided with a pulling protrusion capable of engaging with the tip part of the plunger at the rear end part;
the hub is held by the needle base by a fitting structure formed between the tip part outer peripheral surface of the hub and the inner surface of the tip part of the needle base, and in the fitted state in which the hub is positioned at the closest to the tip side of the needle base, the tip part of the hub is positioned in the vicinity or forward of the tip of the needle base in axial direction.
